# EUROPEAN PATENT APPLICATION

(11) **EP 4 763 066 A1**
(43) Date of publication of application: **24.06.2026**
(21) Application number: 24461651.2
(22) Date of filing: 22.12.2024
(51) Int. Cl.: A61B 5/024, A61B 5/0265, A61B 5/0507, A61B 5/00

(54) **A METHOD FOR DETERMINING TOOTH VITALITY, A DEVICE FOR DETERMINING TOOTH VITALITY AND A COMPUTER PROGRAM PRODUCT THEREFOR**

(71) Applicant: Medisensonic Spolka Akcyjna, 50-382 Wroclaw (PL)
(72) Inventor: Szczepaniak, Zenon, 03-443 Warszawa (PL); Kniola, Michal, 01-471 Warszawa (PL); Pytlarczyk, Lukasz, 01-361 Warszawa (PL)
(74) Representative: Bury & Bury

(57) **Abstract**

The subject of the invention is a method for determining tooth vitality comprising a step of applying radiation and a step of measuring the radiation being the tooth's response to stimulation with radiation to obtain a measurement signal, and then a step of processing the measurement signal to determine time-varying components associated with blood flow to obtain information for assessing tooth vitality. In the step of applying radiation, radio waves with a frequency in the range of 1 to 25 GHz are applied to the tooth. In the step of processing, the time-varying components associated with blood flow are discriminated. The tooth vitality assessment is made on the basis of a comparison of at least one parameter of the components associated with blood flow with a predefined threshold. The subject of the invention is also a measuring device for determining the patient's tooth vitality, comprising a measurement module **(1),** a transceiver **(2)** connected thereto and provided with an arm comprising a transmission line **(4),** and a radiation applicator **(5),** the measurement module being adapted to measure the radiation being the tooth's response to stimulation with radiation to obtain a measurement signal. The transceiver **(2)** is adapted to transmit and receive radiation with a frequency in the range of 1 GHz to 25 GHz. The control module **(10)** is adapted to discriminate time-varying components associated with blood flow. The computer program product according to the invention is adapted to be run in the control module of the device according to the invention and comprises a set of instructions causing the method according to the invention to be performed.

## Description

### Field of the invention

The subject of the invention is a method for determining tooth vitality, a device for determining tooth vitality, and a computer program product for implementing said method for determining tooth vitality using said device.

### State of the art

Dental pulp vitality assessment is based on a complex analysis of the results of the subjective, objective and additional examinations and is necessary in many clinical situations. It is important that the examination of the dental pulp vitality is as minimally invasive as possible.

Typically, minimally invasive examinations use ultrasound, electromagnetic radiation in various bands, including radiation from the visible light range, magnetic resonance imaging and X-ray examinations.

Radio examinations at megahertz and gigahertz frequencies usually have too low resolution and/or too low sensitivity for examining blood flow in the tooth.

In the field of microwave techniques used to measure the flow of various substances, a microwave flowmeter is known from the patent JPH0247521A "Microwave flowmeter". This is a system for measuring the flow of powdered substances through a tube. It uses a microwave generator, a horn antenna and a microwave mixer system. From EP1471826A2, a portable dental device for measuring a tooth is known, however there is no information whether it enables blood flow measurements.

In the prior art, solutions are known relating to the dental pulp vitality examination. Considering the sensitivity and specificity of the examination, the laser Doppler flowmeter is currently considered to be the most effective diagnostic tool for determining the condition of the pulp (H Karayilmaz, Z Kirzioǧlu, Comparison of the reliability of laser Doppler flowmetry, pulse oximetry and electric pulp tester in assessing the pulp vitality of human teeth, J.Oral Rehabil. 2011 May;38(5):340-7. doi: 10.1111/j.1365-2842.2010.02160.x.). The test is semi-quantitative and non-invasive (H. Jafarzadeh, Laser Doppler flowmetry in endodontics: a review, International Endodontic Journal, Volume42, Issue6, June 2009, https:/Idoi.org/10.1111/j.1365-2591.2009.01548.x). Blood flow is detected based on the so-called Doppler effect. Devices of this type are referred to as laser Doppler flowmeters, or LDFs.

Dental tools in the form of forceps with an integrated LDF transmission system are known from documents WO2007072592 and WO2018156722A1. A reflective configuration is also disclosed in WO2018156722A1.

According to numerous studies and publications, the device has a number of limitations. Examination of blood flow in the vessels of the dental pulp using LDF is problematic. During the examination, it is necessary to isolate the tooth in order to eliminate signals from the periodontal tissues and adjacent teeth, and to precisely and stably locate the optical measuring probe. Teeth with fillings and teeth equipped with orthodontic appliances pose a diagnostic problem.

A certain disadvantage of the examination is also the poor repeatability of the results. The variations and measurement errors may be related to many factors: micro-movements of the operator's hand, different force and direction of the probe pressure on the tooth surface, position of the tooth in the arch. Although the laser Doppler flowmeter currently provides the highest diagnostic efficiency in differentiating vital and dead pulp, its use in clinical practice is limited.

Examination of blood flow in the vessels of the dental pulp using LDF is problematic.

### Problem to solve

The prior art has not solved the problem of distinguishing a vital pulp, even after trauma, or a pulp in a state of reversible inflammation from that which must be extirpated in a reliable, safe manner and using a portable device.

### Summary of the invention

The subject of the invention is a method for determining tooth vitality comprising a step of applying radiation and a step of measuring the radiation being the tooth's response to the stimulation with radiation to obtain a measurement signal, and then a step of processing the measurement signal to determine time-varying components associated with blood flow to obtain information for assessing tooth vitality. According to the invention, in the step of applying radiation, radio waves with a frequency in the range of 1 to 25 GHz are applied to the tooth. In the step of processing, the time-varying components associated with blood flow are discriminated. The tooth vitality assessment is made on the basis of a comparison of at least one parameter of the components associated with blood flow with a predefined threshold. The use of microwaves allows one to adjust the penetration depth so that a given tooth can be tested without disturbances from other teeth, even in a reflective configuration. At microwave frequencies, systems can be conveniently constructed and various techniques can be used to obtain information correlated with blood flow in conditions where the Doppler effect associated with blood flow varies during the cardiac cycle and is difficult to detect on the basis of spectral analysis alone. Besides the step of applying radiation, the method according to the invention does not involve affecting the patient's tissues. On the other hand, processing the measurement signal and discriminating components associated with blood flow are technical in nature.

Preferably, in the step of measuring, a synchronization signal representing the patient's blood pulsation and originating from a part of the patient's body other than a tooth is also measured, and in the step of processing, the synchronization signal is used to discriminate time-varying components associated with blood flow. Isolating components associated with blood flow from the signal is much easier if a synchronization signal representing blood flow is available.

Preferably, the measurement signal is subjected to bandpass filtering with a filter with the center frequency set to a frequency determined using the synchronization signal, wherein the filter bandwidth is in the range from 0.1 Hz to 2 Hz.

Preferably, the filter bandwidth is set to 0.2 to 0.4 Hz.

Alternatively, the components associated with blood flow are discriminated by determining a parameter of a cross-correlation function between the measurement signal and the synchronization signal. The cross-correlation function of the synchronization and measurement signals allows one to observe changes in the measurement signal correlated with blood flow and compare them with the values typical for a vital tooth. For example, the maximum of the cross-correlation function or the area under its curve can be used as a criterion.

Preferably, the synchronization signal is obtained using a biopotential sensor, in particular an ECG sensor

Preferably, the synchronization signal is obtained from a pulse oximeter, which is preferably mounted on a finger.

The subject of the invention is also a measuring device for determining the patient's tooth vitality, comprising a measurement module, a transceiver connected thereto and provided with an arm comprising a transmission line, and a radiation applicator, the measurement module being adapted to measure the radiation being the tooth's response to stimulation with radiation to obtain a measurement signal. According to the invention, the transceiver is adapted to transmit and receive radiation with a frequency in the range of 1 GHz to 25 GHz. The device has a control module adapted to discriminate time-varying components associated with blood flow.

Preferably, the transceiver is a microwave generator with self-synchronization by injection.

Preferably, the transceiver is a self-oscillating mixer.

Preferably, the device comprises a sensor connected to the measurement module, adapted to acquire a synchronization signal representing the pulsation of patient's blood in another part of his body, and the control module is adapted to discriminate the time-varying components associated with blood flow based on the synchronization signal from the sensor.

Preferably, the sensor is a biopotential sensor, especially ECG.

Alternatively, the sensor is a pulse oximeter.

The computer program product according to the invention is adapted to be run in the control module of the device according to the invention and comprises a set of instructions for causing the method according to the invention to be performed.

### Advantageous effects of the invention

Microwave measurements in the indicated frequency band are safer for the patient and the doctor than the currently used laser measurements. Due to adjusted penetration depth, they also enable a reflective configuration without the risk of receiving signals from the wrong tooth or other parts of the patient's body. Frequencies above 10 GHz are particularly advantageous in this respect.

The use of an additional synchronization sensor makes it easier to extract the signal associated with blood flow in a vital tooth from the noise signal. As a result, the measurement dynamics and the assessment reliability of whether blood flow in the tooth has been detected, which means that the tooth is alive, or whether it has not been detected, which means that the tooth is dead, are improved. Autocorrelation of the measurement signal at two different microwave frequencies enables similar discrimination.

Thus, the invention according to this application solves the problem of assessing blood flow in the dental pulp in a non-invasive manner. The system according to the invention measures the velocity of blood flow in the blood vessels of the dental pulp using the Doppler effect, similarly to known laser solutions. However, an electromagnetic wave from the microwave frequency range is used as the probing radiation. The wave frequency is selected so as to obtain the wave penetration into the tooth tissue smaller than the size (thickness) of the tooth in the direction of wave propagation, and the correlation of the measurement signal with the synchronization signal representing blood pulsation enables sufficient differentiation of the useful signal from noise and interference.

For electromagnetic waves from the microwave range, the difference in the electrical permittivity of dentine and pulp (e.g. at 10 GHz, the electrical permittivity of dentine is 12 compared to the electrical permittivity of pulp of 30-40) causes greater signal reflection at the dentine-pulp boundary and lower total transmission through the tooth structure.

It also turned out unexpectedly that, in comparison to LDF, the device according to the invention is less sensitive to disturbance related to fillings, crowns, color changes due to dirt, injuries or organic causes, and the variability of parameters due to ethnic factors.

### Description of the drawing

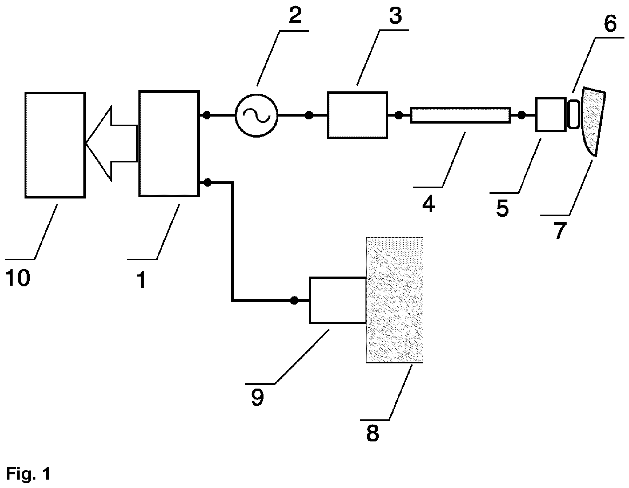

The subject of the invention has been shown in embodiments in the drawing, in which Fig. 1 shows a schematic diagram of a system according to an embodiment of the invention.

### Description of the embodiments of the invention

In the first embodiment of the method according to the invention, radiation in the form of an electromagnetic field with a frequency in the range from 2 GHz to 25 GHz is directed at the tooth. As a result of directing radiation at the tooth, the tooth generates a response in the form of reflected radiation or transmitted radiation. The properties of the reflected and transmitted radiation depend on whether and how much blood is in the tooth, i.e. on blood supply. The blood supply to the tooth changes in the rhythm of the patient's pulse (heartbeat). Moreover, the movement of blood generates Doppler components in the reflected radiation and in the transmitted radiation, shifted in frequency due to the pulsatile movement of the blood. The method according to the invention is based on the reception and processing of this radiation originating from the patient's stimulated tooth.

To implement the method according to the invention, it is convenient to use a measuring device according to an embodiment of the invention. The schematic diagram of the device according to an embodiment of the invention is shown in Fig. 1. The device comprises a measurement module **1,** a transceiver **2** connected thereto and provided with an extended arm comprising a transmission line **4,** and a radiation applicator **5.** Preferably, the device further comprises a sensor **9,** connectable to the measurement module **1,** adapted to acquire a synchronization signal representing the patient's blood pulsation. In the absence of this sensor, discrimination of the time-varying components associated with blood flow in the tooth is determined based on the autocorrelation of the measurement signal.

In the present embodiment, the synchronization signal sensor **9** is a pulse oximeter or other photoplethysmographic device. However, the sensor **9** can be implemented in various ways, e.g. as an ECG sensor or other biopotential sensor, in particular mounted on a finger. It is important that the signal from the sensor carries information about the patient's blood pulsation in a place which is certainly properly supplied with blood. In the present embodiment, the sensor **9** is mounted on a finger, which is convenient for the patient. ECG electrodes attached to the chest may also be used.

The measurement module **1** is adapted to obtain the measurement signal from the transceiver **2** and the synchronization signal from the sensor **9.** The measurement module **1** transmits the measurement signal and the synchronization signal to the control module **10.** In the present embodiment, the measurement module **1** consists of an analog-to-digital converter and a microprocessor, a power supply system and a measurement information visualization system.

During the measurement of the examined tooth **7,** it is best to use a buffer layer **6** which is in contact with the radiation applicator **5** - in the present embodiment, the applicator is a section of a microstrip line 3 mm long and 0.7 mm wide formed on a substrate made of a glass-Teflon laminate 0.7 mm thick and 5 mm x 5 mm in size. The thickness of the copper metallization of the microstrip line is 36 µm. The radiation reflected from the tooth **7** returns to the measurement module **1** via the applicator, and the Doppler effect resulting from the contact of the radiation with the flowing blood affects the measurement signal.

The optional buffer layer **6** is made of light-cured Gradia Direct with a thickness of 0.5 mm and 5 mm x 5 mm in size. Between the input of the radiation applicator **5** and the output of the transceiver **2,** a wave transmission system **4** is introduced in the form of a coaxial line section with a length of 30 mm, characteristic impedance of 50 Ω, and external diameter of 3 mm. This system is connected to the radiation applicator **5** on the ground plane metallization side of the microstrip line through an opening in the metallization and the substrate. On the other side, this system is connected to a matching circuit **3** in the form of a microstrip line segment 5.1 mm long and 3 mm wide, made on a glass-Teflon substrate 0.7 mm thick and with a relative permittivity of 2.5.

The control module **10** is adapted to process the measured signals and to control the transceiver. The control module **10** is essentially a computer performing the method according to the invention. The control module **10** can be implemented in various ways, e.g. as a microcontroller, a microcomputer, a dedicated AISIC, as a distributed system, or as a unit containing components implemented in whole or in part in software on various hardware components. The control module **10** is adapted to control the measurement module **1,** to receive and process the measurement and synchronization signals from the measurement module, and to compare an amplitude of the spectrum of the measurement signal around the blood pulsation frequency determined using the synchronization signal from the sensor **9** in a band having a width ranging from 0.1 Hz to 2 Hz, with a predefined threshold value. In particular, the measurement module and the control module can be integrated in one device.

The transceiver **2** is adapted to transmit and receive radiation with a frequency in the range from 1 GHz to 25 GHz. The transceiver **2** is implemented as a self-oscillating mixer with an FHX13LG transistor generating a microwave signal with a frequency of 10 GHz and a power of 5 mW. The signal reflected from the tooth returns to the self-oscillating mixer and affects its operating conditions. As a result, an intermediate frequency signal containing a time-varying component dependent on the Doppler effect caused by blood flow in the tooth and also with time-varying intensity dependent on changes in tissue parameters due to changes in blood supply is received from the mixer. In dead teeth, in which no blood flows, the aforementioned phenomena do not occur, therefore it is possible to determine the parameter of the time-varying components associated with blood flow and the threshold value of this parameter.

In alternative embodiments, transceivers are used comprising separate transmitting and receiving paths connected using a microwave circulator, as well as a transceiver in the form of a microwave generator with self-synchronization. The latter proved to be particularly advantageous.

In the step of applying radiation of the method according to the invention, the tested tooth **7** is covered with the buffer layer **6** of light-cured composite material Gradia Direct with a thickness of 0.5 mm and 5 mm x 5 mm in size, and then stimulated with an electromagnetic field with a frequency of 10 GHz and a power of 5 mW by bringing closer the radiation applicator **5** placed on the boom, fed from the fifty-ohm transceiver **2** via the matching circuit **3** and the wave transmission system **4.** The electromagnetic wave leaving the matching circuit **3** enters the wave transmission system **4** and propagates further to the radiation applicator **5.** The wave propagating in the structure of the applicator **5** based on a planar transmission line has an electric field distribution extending beyond the substrate of the line, due to which the electric field penetrates the material of the buffer layer **6** and the tooth tissue **7.**

A part of the wave that penetrates the tooth tissue **7,** i.e. enamel, dentine, reaches the dental pulp, where it is dispersed and reflected due to the difference in the electrical permittivity of the pulp and the dentine.

The reflected wave undergoes a Doppler shift and its frequency is modulated by the pulsatile motion of blood in the pulp blood vessels. The modulating frequency ranges from 0.5 Hz to 3 Hz depending on the patient's heart rate during the examination.

In the step of measuring the radiation being the tooth's response to stimulation with radiation, a measurement signal is obtained. The form and shape of the measurement signal depend on the receiver or transceiver used. It is important that the impact of the Doppler effect resulting from blood flow on the radiation received from the tooth in response to stimulation can be identified and analyzed in this signal.

A part of the wave power reflected from the tooth **7** and modulated due to reflection returns through the radiation applicator **5,** the wave transmission system **4** and the matching circuit **3** back to the transceiver **2.** Then, it is subjected to the process of frequency conversion and phase detection in the microwave generator system with self-synchronization by injection. The time-varying component of the measurement signal resulting from the Doppler effect is frequency-modulated with a deviation dependent on the changes in the blood flow velocity and a modulating frequency equal to the heartbeat frequency. The time-varying component is further processed in the detection circuit where the instantaneous frequency changes are converted to changes in the signal amplitude. As a result, a time-varying signal with an intermediate frequency equal to the frequency of the pulsating blood movement is obtained.

The synchronization signal with the blood pulsation frequency is obtained directly from the sensor **9.** In the absence of the sensor, the autocorrelation function can be used on the measurement signal lasting at least several heartbeats. The measurement signal correlation function works better if the measurement is carried out at different frequencies.

In the step of processing the measurement signal, that was received and converted to an intermediate frequency, the power in the frequency band associated with the pulsating blood flow is determined so as to obtain information for assessing the tooth vitality.

The output signal of the sensor **9** is converted to digital form in the measurement module and used in the algorithms for processing the output signal of the transceiver **2.**

The heartbeat frequency is determined from the output signal of the sensor **9** by calculating the time difference between the signal maxima in time domain or by calculating the Fourier transform and determining the dominant frequency component. Information about the heartbeat frequency is further used to define a band-pass filter with a center frequency equal to the heartbeat frequency and a passband width of 0.1 to 2 Hz. Good effects were obtained for filters with a width equal to 20% of the heart rate.

Then the passband frequency range of the filter is used as the integration (calculation) range of the power of the signal from transceiver **2.** The value obtained in this way is a parameter dependent on the degree of blood flow in the dental pulp.

The amplitude summed in the band with the center frequency corresponding to the blood pulsation and the defined width, preferably 20%, after filtration is a measure of the blood flow in the tested tissue. Comparison of this amplitude with the reference value allows one to determine whether blood is flowing in the tooth, which means that it is vital, or not, which means that it is dead.

The reference value can be determined based on the population or by measuring another tooth - preferably symmetrical to the tested one. The tested tooth, if vital, should not show an amplitude lower than 25% of the amplitude obtained for the vital tooth.

Filters of different widths were tested. Generally, band-pass filters with a width ranging from 0.1 Hz to 2 Hz worked well. Unexpectedly, by setting the center frequency of the filter to the blood pulsation frequency, filters with narrow passbands worked better. The best results were obtained for the 0.2-0.4 Hz band centered around the pulsation frequency.

As a result, the tooth vitality assessment is made based on a comparison between the predefined threshold and the spectrum amplitude of the Doppler component of the measurement signal around the blood pulsation frequency in a band having a width ranging from 0.1 Hz to 2 Hz, determined using the synchronization signal.

In an alternative embodiment, the time-domain form of the output signal of the sensor **9** is used as a reference and the cross-correlation function of the time-domain signals from the sensor **9** and the transceiver **2** is determined. Then, parameters in the form of the maximum of the cross-correlation function or the area under its curve are used as carrying information about the blood flow in the dental pulp.

For a dead tooth, the cross-correlation function exhibits very small values. The correlation parameters of the synchronization and measurement signals are a good measure of the similarity of the reference and measurement signals. If the correlation function has strong maxima, it means that in the microwave signal there is the same pulsating component as is present in the reference signal - and therefore blood is flowing in the tooth.

In the microwave device according to the invention, the frequency of the emitted wave can be adjusted so as to obtain the optimal penetration depth into the tooth tissue, no greater than the tooth dimension along the direction of wave propagation. For electromagnetic waves from the microwave range, the so-called penetration depth, means the distance into the medium along the wave propagation path where the power drops to 1/e² of the initial power.

In the frequency range from 8 to 22 GHz, the penetration depth of an electromagnetic wave into the tooth material, i.e. tissue and bone, is comparable to the dimensions of the tooth, which reduces the risk of wave transmission to tissues adjacent to the tested tooth and thus obtaining a distorted measurement result.

For example, at a frequency of 10 GHz, the penetration depth of an electromagnetic wave into the tissue is 2.8 mm, while the penetration depth of an electromagnetic wave into the bone is 7.2 mm. The 8 to 12 GHz range is particularly well suited for examining large molars.

At a frequency of 20 GHz, the penetration depth of an electromagnetic wave into the tissue is 1.2 mm and the penetration depth of an electromagnetic wave into the bone is 3.4 mm - 1.2 mm. The range from 18 to 22 GHz is particularly well suited for examining smaller teeth.

An additional speculum or imaging device can be integrated into the arm of the measuring device to facilitate precise application.

### Industrial applicability of the invention

The basic function of the invention is to image blood flow in the vessels of the dental pulp, which allows one to determine the tooth's condition. The parameter defining the tooth vitality is the confirmation of blood flow in the pulp. Assessment of the pulp condition is a significant diagnostic problem encountered during dental treatment.

The device according to the invention can be used in all procedures in which it is crucial to diagnose the dental pulp vitality: endodontics, implantology, prosthetic procedures, an element of orthodontic treatment. The scope of use of the device is both objective and subjective due to the elimination of disturbance related to fillings, crowns, color changes due to dirt, injuries or organic causes, and the variability of parameters due to ethnic factors.

From the perspective of treatment, the invention solves the key problem of distinguishing the vital pulp, even after trauma, or a pulp in a state of reversible inflammation from that which must be extirpated. Until recently, the detection of pulp necrosis or irreversible inflammation resulted in the need to implement root canal (endodontic) treatment. Endodontic procedures have a significant impact on the biomechanical properties of tooth tissues, and tooth fractures after root canal treatment are the third most common cause of tooth loss, after caries and periodontal disease. A tooth after root canal treatment has limited amount of hard tissue, is more fragile and susceptible to fracture. Activities related to obtaining access to the canals, i.e. removal of the tooth chamber and widening the part of the canal adjacent to the chamber, result in the loss of approx. 25% of the tooth's hard tissue. The use of medical agents reduces the microhardness of dentin by 20-27%, and the use of chelating agents by 50%.

The pulp vitality assessment is based on a complex analysis of the results of the subjective, objective and additional examinations and is necessary in many clinical situations.

The scope of application of the device is not limited to teeth. Tests have shown that the device can be used to detect inflammatory foci in oncological patients who suffer from impaired blood supply to the head and neck tissues after radiotherapy of the head and neck area, chemotherapy.

The invention enables characterizing blood flow with parameters directly reflecting the speed and variability of blood flow over time. The device according to the invention measures the Doppler frequency shift resulting from blood flow and the pulsation frequency (HR) resulting from the periodic work of the heart. As a measure of perfusion, it is possible to use combined information in the form of: signal amplitude at the HR frequency, signal amplitude at the HR frequency measured in the passband in the frequency domain, and the cross-correlation function of the reference and measurement signal determined in the time domain. Currently measured values of the above parameters are compared with the reference values determined as a result of clinical tests at the device calibration step.

The advantage of the invention is also a smaller penetration depth of the radiation into the tissue and a smaller total transmission through the tooth structure compared to LDF solutions.

The invention reduces the susceptibility of measurement to artifacts related to mechanical movements and vibrations.

The invention provides the possibility of effective examination of teeth with fillings, crowns, teeth equipped with orthodontic appliance elements. Unlike laser solutions, the invention ensures insensitivity to the color of the enamel. Also, a change in the parameters of the enamel material due to dirt or organic causes does not significantly affect the transmission possibilities.

A person skilled in the art is able to routinely propose various adequate transceivers **2** and select their impedances according to the impedance of the radiation applicator **5** or the transmission line. The use of the buffer layer **6** is optional, although beneficial. A person skilled in the art is also able to routinely propose various structures of radiation applicators.

## Claims

1. A method for determining tooth vitality, comprising a step of applying radiation and a step of measuring radiation being the tooth's response to the stimulation with the radiation, to obtain a measurement signal, and then a step of processing the measurement signal to determine time-varying components associated with blood flow to obtain information for assessing tooth vitality, **characterized in that**
in the step of applying the radiation, radio waves with a frequency in the range of 1 to 25 GHz are applied to the tooth, and
in the step of processing, the time-varying components associated with blood flow are discriminated,
wherein the tooth vitality assessment is made on the basis of a comparison of at least one parameter of the components associated with blood flow with a predefined threshold.

2. The method according to claim 1, wherein in the step of measuring, a synchronization signal representing the patient's blood pulsation and originating from a part of the patient's body other than a tooth is also measured, and in the step of processing, the synchronization signal is used to discriminate time-varying components associated with blood flow.

3. The method according to claim 2, **characterized in that** the measurement signal is subjected to bandpass filtering with a filter having a center frequency set to a frequency determined using the synchronization signal, wherein the filter bandwidth is set to a width in a range of 0.1 Hz to 2 Hz.

4. The method according to claim 3, wherein the filter bandwidth is set to 0.2 to 0.4 Hz.

5. The method according to claim 2, wherein the components associated with blood flow are discriminated by determining a parameter of a cross-correlation function between the measurement signal and the synchronization signal.

6. The method according to any one of claims 1 to 5, wherein the synchronization signal is obtained using a biopotential sensor.

7. The method according to any one of claims 1 to 5, wherein the synchronization signal is obtained from a pulse oximeter.

8. The method according to claim 7, wherein the pulse oximeter is mounted on a finger.

9. A measuring device for determining the patient's tooth vitality, comprising a measurement module (1), a transceiver (2) connected thereto and provided with an arm comprising a transmission line **(4),** and a radiation applicator **(5),** the measurement module being adapted to measure the radiation being the tooth's response to stimulation with radiation to obtain a measurement signal, **characterized in that** the transceiver **(2)** is adapted to transmit and receive radiation with a frequency in the range of 1 GHz to 25 GHz, and the device has a control module **(10)** is adapted to discriminate time-varying components associated with blood flow.

10. The device according to claim 9, wherein the transceiver is a microwave generator with self-synchronization by injection.

11. The device according to claim 9, wherein the transceiver is a self-oscillating mixer.

12. The device according to any one of claims from 9 to 11, **characterized in that** it comprises a sensor **(9)** connected to the measurement module **(1)** adapted to acquire a synchronization signal representing the pulsation of patient's blood in another part of his body, and the control module **(10)** is adapted to discriminate the time-varying components associated with blood flow based on the synchronization signal from the sensor **(9).**

13. The device according to any one of claims 9 to 12, wherein the sensor **(9)** is a biopotential sensor.

14. The device according to any one of claims 9 to 12, wherein the sensor **(9)** is a pulse oximeter.

15. A computer program product adapted to be run in the control module **(10)** of the device as defined in any one of claims 9-14, comprising a set of instructions for causing the method as defined in any one of claims 1 to 8 to be performed.
